(19) Europäisches Patentamt
European Patent Office
Office européen des brevets

(11) Veröffentlichungsnummer: **0 519 942 B1**

(12) **EUROPÄISCHE PATENTSCHRIFT**

(45) Veröffentlichungstag der Patentschrift: **05.10.94**

(51) Int. Cl.⁵: **A61K 7/50**, A61K 7/08, C11D 1/94

(21) Anmeldenummer: **91905070.8**

(22) Anmeldetag: **26.02.91**

(86) Internationale Anmeldenummer: **PCT/DE91/00163**

(87) Internationale Veröffentlichungsnummer: **WO 91/13610 (19.09.91 91/22)**

# (54) KOSMETISCHE REINIGUNGSMITTEL.

(30) Priorität: **10.03.90 DE 4007638**

(43) Veröffentlichungstag der Anmeldung: **30.12.92 Patentblatt 92/53**

(45) Bekanntmachung des Hinweises auf die Patenterteilung: **05.10.94 Patentblatt 94/40**

(84) Benannte Vertragsstaaten: **AT BE CH DE DK ES FR GB GR IT LI LU NL SE**

(56) Entgegenhaltungen:
**GB-A- 2 114 996**
**GB-A- 2 203 444**

(73) Patentinhaber: **Beiersdorf Aktiengesellschaft**
**Unnastrasse 48**
**D-20245 Hamburg (DE)**

(72) Erfinder: **SCHÖNROCK, Uwe**
**Mittelstrasse 18 b**
**D-2000 Norderstedt (DE)**
Erfinder: **LERG, Heike**
**Glindweg 15**
**D-2000 Hamburg 60 (DE)**
Erfinder: **PAPE, Wolfgang**
**Lydiastrasse 3**
**D-2000 Hamburg 70 (DE)**
Erfinder: **STEIGER, Klaus-Michael**
**Oberstrasse 108**
**D-2000 Hamburg 13 (DE)**
Erfinder: **OSTHOF, Petra**
**Beim Oberstein 30**
**D-2000 Hamburg 54 (DE)**
Erfinder: **GERLACH, Kerstin**
**Gro heidestr. 1a**
**D-2000 Hamburg 60 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft kosmetische Reinigungsmittel.

Derartige Mittel sind an sich bekannt. Es handelt sich dabei im wesentlichen um oberflächenaktive Substanzen oder Stoffgemische, die dem Verbraucher in verschiedenen Zubereitungen angeboten werden.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark.

Das gebräuchlichste Tensid für kosmetische Zusammensetzungen ist das Natriumlaurylethersulfat. Obwohl es gute Waschkraft besitzt und einigermaßen haut- und Schleimhautverträglich ist, sollten empfindliche Personen den häufigen Kontakt damit meiden.

Es ist zwar eine große Zahl recht milder Tenside erhältlich, jedoch sind die Tenside des Standes der Technik entweder mild, reinigen aber schlecht, oder aber sie reinigen gut, reizen jedoch Haut oder Schleimhäute.

Es war somit die Aufgabe der vorliegenden Erfindung, den Mißständen des Standes der Technik abzuhelfen.

Erfindungsgemäß wird diese Aufgabe gelöst durch ein Reinigungsmittel, enthaltend

2 - 10 Gew.-% eines oder mehrerer Amidoamin-amphoterer Tenside

0,5 - 15 Gew.-% eines oder mehrerer Sulfoacetat-Tenside und

gegebenenfalls eines oder mehrerer der Tenside, gewählt aus der Gruppe der Sarcosinate, der Betaine, der Sulfobetaine, der Amidosulfobetaine, der Sulfosuccinate und der ethoxylierten Alkoholcarboxylate.

Es ist an sich bekannt, die beiden einzelnen Hauptbestandteile der erfindungsgemäßen Zusammensetzungen, Amidoamin-amphotere Tenside und Sulfoacetat-Tenside, in kosmetischen Reinigungsmitteln einzusetzen.

Es ist auch bekannt, die einzelnen Hauptbestandteile mit anderen oberflächenaktiven Substanzen zu mischen. Es konnte aber nicht vorhergesehen werden, daß die Kombination der erfindinngsgemäßen Hauptbestandteile zu Produkten führen würde, die sich gegenüber den Einzelsubstanzen durch bessere Verträglichkeit mit Haut und Schleimhäuten auszeichnen und zudem überlegene Waschkraft aufweisen. Demnach liegt ein echter Synergismus vor.

Es konnte weiterhin überraschend festgestellt werden, daß dieser synergistische Effekt unterstützt wird, wenn die erfindungsgemäßen Zusammensetzungen Tenside aus der Gruppe der Sulfosuccinate, der Betaine, der Sarcosinate und/oder der carboxylierten Alkoholethoxylate enthalten.

Besonders überraschend war, daß die erfindinngsgemäßen Zusammensetzungen bei der Anwendung einen äußerst feinblasigen, cremigen Schaum ergeben.

Die Hauptbestandteile sind wie folgt charakterisiert:

Unter Amidoamin-amphoteren Tensiden werden Verbindungen der folgenden allgemeinen Formeln verstanden:

a) 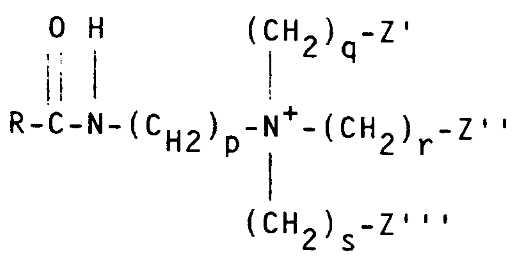

wobei

| | |
|---|---|
| R | einen gesättigten Kohlenwasserstoffrest mit 6 - 25 C-Atomen bedeutet, |
| Z', Z'' | unabhängig gewählt werden aus der Gruppe OH , $-COO^- M^+$ , |
| Z''' | $-COO^-$ bedeutet, |
| $M^+$ | ein Kation, gewählt aus der Gruppe |

$H^+$, $Na^+$, $K^+$, $NH_4{}^+$, $H_mN^+(-CH_2-CH_2-OH)_{4-m}$ , $H_mN^+(-CH_2-CH_3)_{4-m}$ oder $H_mN^+(CH_3)_{4-m}$ bedeutet,

p , q , r , s unabhängig voneinander Zahlen von 1 - 5 bedeuten,

m 0, 1, 2 oder 3 bedeutet.

b) 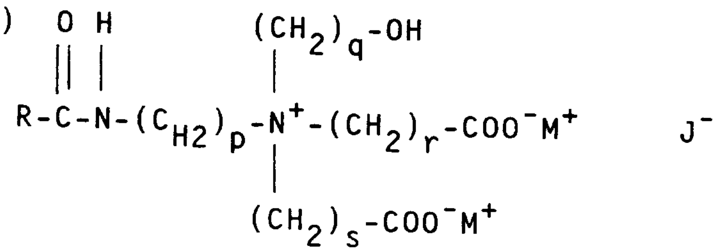

wobei

R einen gesättigten Kohlenwasserstoffrest mit 6 - 25 C-Atomen bedeutet,

$M^+$ ein Kation, gewählt aus der Gruppe $H^+$, $Na^+$, $K^+$, $NH_4{}^+$, $H_mN^+(-CH_2-CH_2-OH)_{4-m}$ , $H_mN^+(-CH_2-CH_3)_{4-m}$ oder $H_mN^+(CH_3)_{4-m}$ bedeutet,

p , q , r , s unabhängig voneinander Zahlen von 1 - 5 bedeuten,

m 0, 1, 2 oder 3 bedeutet,

$J^-$ ein Anion , gewählt aus der Gruppe Laurylsulfat, Laurylethersulfat bedeutet.

c) 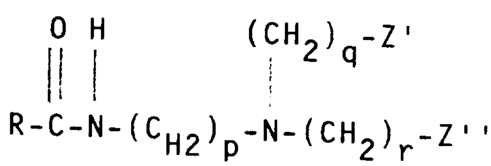

wobei

R einen gesättigten Kohlenwasserstoffrest mit 6 - 25 C-Atomen bedeutet,

Z', Z'' unabhängig gewählt werden aus der Gruppe OH , $-COO^-$ $M^+$ ,

$M^+$ ein Kation, gewählt aus der Gruppe $H^+$, $Na^+$, $K^+$, $NH_4{}^+$, $H_mN^+(-CH_2-CH_2-OH)_{4-m}$ , $H_mN^+(-CH_2-CH_3)_{4-m}$ oder $H_mN^+(CH_3)_{4-m}$ bedeutet,

p , q , r unabhängig voneinander Zahlen von 1 - 5 bedeuten,

m 0, 1, 2 oder 3 bedeutet.

Unter Sulfoacetaten werden im folgenden Verbindungen der allgemeinen Formel

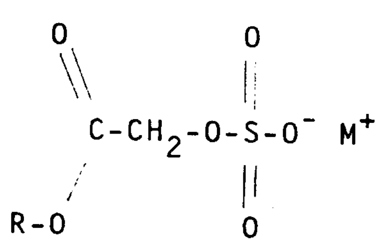

verstanden, wobei

R einen gesättigten Kohlenwasserstoffrest mit 6 - 25 C-Atomen bedeutet,

$M^+$ ein Kation, gewählt aus der Gruppe $H^+$, $Na^+$, $K^+$, $NH_4{}^+$, $H_mN^+(-CH_2-CH_2-OH)_{4-m}$ , $H_m(-CH_2-CH_3)_{4-m}$ oder $H_m(CH_3)_{4-m}$ bedeutet,

m 0, 1, 2 oder 3 bedeutet.

Die Nebenbestandteile sind wie folgt charakterisiert:
Unter Sulfosuccinaten werden Verbindungen der allgemeinen Formel

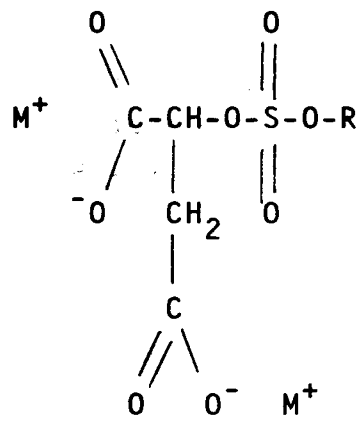

verstanden.

Unter Betainen werden Verbindungen der allgemeinen Formel

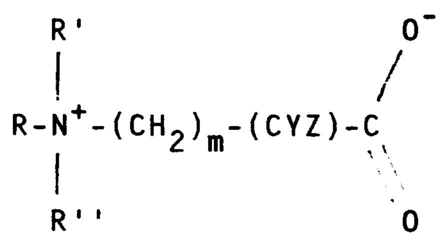

verstanden.

Unter Sulfobetainen werden Verbindungen der allgemeinen Formel

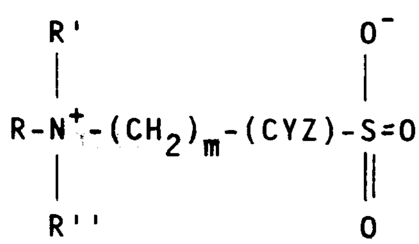

verstanden.

Unter Amidosulfobetainen werden Verbindungen der allgemeinen Formel

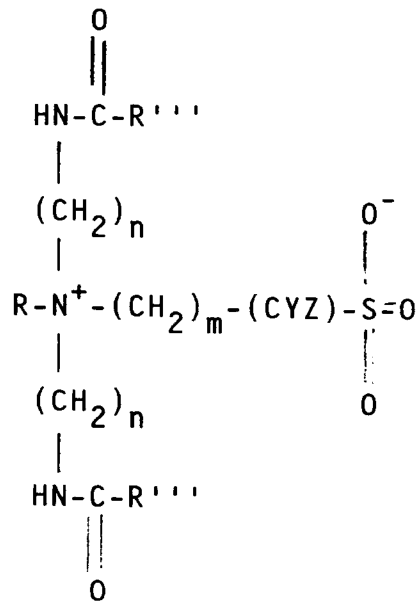

verstanden.

Unter Sarcosinaten werden Verbindungen der allgemeinen Formel

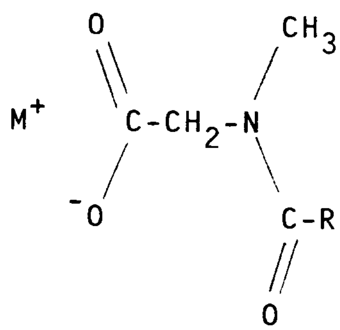

verstanden.

Unter ethoxylierten Alkoholcarboxylaten werden Verbindungen der allgemeinen Formel

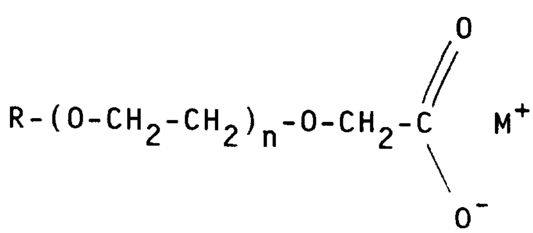

verstanden.

Für die Nebenbestandteile bedeutet

| | |
|---|---|
| R | einen gesättigten Kohlenwasserstoffrest mit 6 - 25 C-Atomen, |
| R', R'' und R''' | werden unabhängig voneinander aus der Gruppe H, $-CH_2-CH_2-OH$ , $-(CH_2)_2COO^-$ $M^+$ gewählt, |
| Y, Z | werden unabhängig voneinander gewählt aus der Gruppe H, Methyl |
| $M^+$ | bedeutet ein Kation, gewählt aus der Gruppe $H^+$, $Na^+$, $K^+$, $NH_4{}^+$, $H_mN^+(-CH_2-CH_2-OH)_{4-m}$ , $H_mN^+(-CH_2-CH_3)_{4-m}$ oder $H_mN^+(CH_3)$- |

4-m ,

n bedeutet eine Zahl von 1 - 5 ,

m bedeutet 0, 1, 2 oder 3.

Bevorzugt enthalten die erfindungsgemäßen Reinigungsmittel

2 - 10 Gew.-% eines oder mehrerer Amidoamin-amphoterer Tenside

0,5 - 15 Gew.-% eines oder mehrerer Sulfoacetate und

0,5 - 8 Gew.-% eines oder mehrerer Sulfosuccinate,

5 - 15 Gew.-% eines oder mehrerer Betaine

2 - 20 Gew.-% eines oder mehrerer Sarcosinate

10 - 20 Gew.-% eines oder mehrerer carboxylierter Alkoholethoxylate,

bezogen auf das Gesamtgewicht der Zusammensetzung. Besonders bevorzugt enthalten die erfindungsgemäßen Reinigungsmittel

2 - 8 Gew.-% eines oder mehrerer Amidoamin amphoterer Tenside

0,5 - 5 Gew.-% eines oder mehrerer Sulfoacetate und

0,5 - 5 Gew.-% eines oder mehrerer Sulfosuccinate,

5 - 12 Gew.-% eines oder mehrerer Betaine

2 - 10 Gew.-% eines oder mehrerer Sarcosinate

10 - 15 Gew.-% eines oder mehrerer carboxylierter Alkoholethoxylate,

bezogen auf das Gesamtgewicht der Zusammensetzung. Dabei sollte die Gesamtmenge der Tenside höchstens 50 Gew.-% betragen.

Vorteilhaft werden die erfindungsgemäßen Amidoaminamphoteren Tenside gewählt aus der Gruppe die nach der CTFA-Nomenklatur die Bezeichnung Alkylamphodiacetate, Alkylamphoacetate, Alkylamphodipropionate sowie Alkylamphopropionate tragen.

Besonders vorteilhaft werden die erfindungsgemäßen Amidoamin amphoteren Tenside gewählt aus der Gruppe, die nach der CTFA-Nomenklatur die Bezeichnungen

Cocoamphodiacetate, Cocoamphoacetate, Cocoamphopropionate, Cocoamphodipropionate, Lauroamphodiacetate, Lauroamphoacetate, Lauroamphodipropionate, Caproamphodiacetate, Caproamphoacetate, Capryloamphodiacetate, Capryloamphoacetate, Capryloamphodipropionate, Stearoamphodiacetate, Stearoamphoacetate, Isostearoamphoacetate, Ispstearoamphodipropionate

tragen.

Die Sulfoacetat-Tenside werden bevorzugt gewählt aus der Gruppe

Laurylsulfoacetat, Cocoylsulfoacetat, Oleylsulfoacetat, Stearylsulfoacetat und Isostearylsulfoacetat.

Die Betaine werden bevorzugt gewählt aus der Gruppe der höheren Alkylbetaine, der Sulfobetaine oder der Amidosulfobetaine, insbesondere der Gruppe aus

Coco-dimethyl-carboxymethyl-betain, Lauryl-carboxymethyl-betain, Lauryl-dimethyl-(alpha-carboxyethyl)-betain, Cetyl-dimethyl-carboxymethyl-betain, Lauryl-(bis-(2-hydroxyethyl))-carboxymethyl-betain, Stearyl-(bis-(2-hydroxypropyl))-carboxymethyl-betain, Oleyl-dimethyl-(gamma-carboxypropyl)-betain, Lauryl-(bis-(2-hydroxypropyl)-(alpha-carboxymethyl)-betain, Coco-dimethyl-sulfodimethyl-sulfoethyl-betain, Lauryl-(bis-(2-hydroxyethyl))-sulfopropyl-betain, Coco-amidopropyl-betain, Cocobetain, Myristyl-amidopropyl-betain.

Die Sulfosuccinate werden bevorzugt gewählt aus der Gruppe der N-acyl-sulfosuccinate, bevorzugt gewählt aus der Gruppe

der Lauryl-sulfosuccinate, der Cocoyl-sulfosuccinate, der Oleyl-sulfosuccinate, der Stearyl-sulfosuccinate, der Isostearyl-sulfosuccinate.

Die carboxylierten Alkoholethoxylate werden bevorzugt gewählt aus der Gruppe

Natrium-cocoylcarboxyamid-(MEA)-carboxylat, Natrium-cocoylcarboxyamid-(MEA)-carboxylat, Natrium-lauroylcarboxyamid-(MEA)-carboxylat, Natrium-lauroylcarboxyamid-(MEA)-carboxylat. Dabei bedeutet MEA Monoethanolammonium

($H_3N^+(-CH_2-CH_2-OH)$ ).

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, Perlglanzagentien, Pflanzenextrakte, Vitamine, Wirkstoffe und dergleichen.

Tabelle 1

| Beispiel | 1 | 2 | 3 | 4 | 5 |
|---|---|---|---|---|---|
| Lauroamphocarboxy-glycinat (Rewoteric AM2L, REWO Chem.Group) | 5,52 | 8,94 | 6,58 | 5,52 | 3,94 |
| Natrium-laurylsulfo-acetat (Lanthanal LAL, Stepan Comp.) | 3,00 | 4,86 | 3,58 | 2,00 | 1,42 |
| Natrium-lauroylsarcosinat (Medialan LD30, Hoechst Celanese Corp.) | 13,34 | - | - | - | - |
| Cocoamidopropylbetain (Tego Betaine L7, Goldschmidt Chem.Group) | 7,66 | 12,34 | 10,00 | 7,66 | 6,00 |
| Natrium-coco-carboxyl-amid MEA-4 carboxylat (Akypo Soft KA250BV,Chemy) | - | - | - | 15,66 | 13,34 |
| Natrium-lauryl-sulfo-succinat (Rewopal SBFA 30, REWO Chem. Group) | - | - | 6,26 | - | 5,50 |
| Natriumlaurylethersul-fat 27,5 , Henkel KGaA | - | - | - | - | - |
| PEG-7-glycerylcocoat (Cetiol HE, Henkel KGaA) | 1,00 | 1,00 | 1,00 | 1,00 | 1,00 |
| Glycol-distearat (Tegin G1100, Goldschmidt Chem.Group) | 0,50 | 0,50 | 0,50 | 0,50 | 0,50 |

Fortsetzung Tabelle 1

| | | | | | |
|---|---|---|---|---|---|
| Parfüm | 0,40 | 0,40 | 0,40 | 0,40 | 0,40 |
| Phenoxyethanol, Methyl-, Ethyl-, Propyl- und Butylparaben (Phenonip, Nipa Lab. Inc.) | 0,30 | 0,30 | 0,30 | 0,30 | 0,30 |
| PEG-200 Glycerinmonofettsäureester (Rewoderm LI-420-70, Rewo Chem. Group) | 4,60 | - | - | 1,60 | 3,60 |
| NaCl | - | - | - | - | - |
| Zitronensäure | 0,16 | 0,16 | 0,12 | 0,12 | 0,12 |
| Wasser | 63,52 | 71,50 | 71,26 | 65,24 | 63,88 |

Die erfindungsgemäßen Zusammensetzungen gemäß den Beispielen 1 - 6 werden wie folgt hergestellt: Die Tenside, das PEG-7-glycerylcocoat, das Perlglanzagens (Glycol-distearat) und etwa 10 Gew.-% Wasser werden unter Rühren auf 70 °C erhitzt und mit Natriumlaurylsulfoacetat versetzt. Das Reaktionsgemisch wird langsam auf Raumtemperatur abgekühlt, so daß das Perlglanzagens auskristallisieren kann. Zitronensäure und Natriumchlorid werden in einer kleinen Menge Wasser aufgelöst und zu dem Gemisch gegeben. Zuletzt werden das Konservierungsmittel, das Parfüm und das Verdickungsmittel sowie das restliche Wasser zugegeben und die Zusammensetzung homogenisiert.

**Patentansprüche**

**1.** Reinigungsmittel, bevorzugt für kosmetische Zwecke, enthaltend
2 - 10 Gew.-% eines oder mehrerer Amidoamin-amphoterer Tenside
0,5 - 15 Gew.-% eines oder mehrerer Sulfoacetat-Tenside und
gegebenenfalls eines oder mehrere der Tenside gewählt aus der Gruppe der Sulfosuccinate der allgemeinen Formel

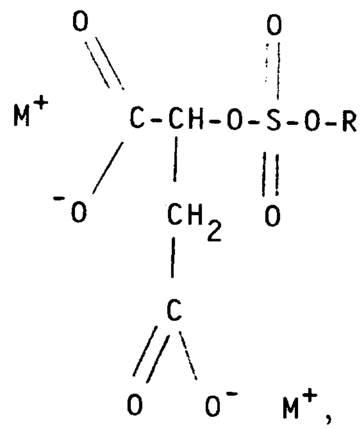

der Betaine der allgemeinen Formel

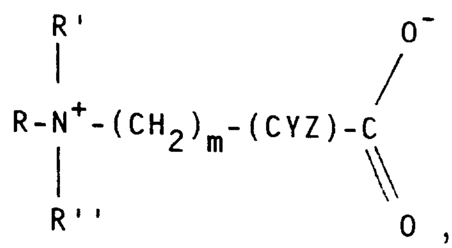

der Sulfobetaine der allgemeinen Formel

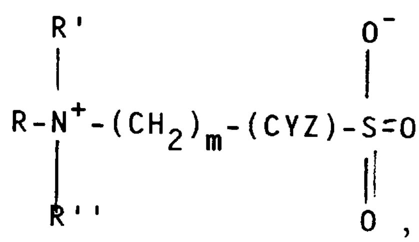

der Amidosulfobetaine der allgemeinen Formel

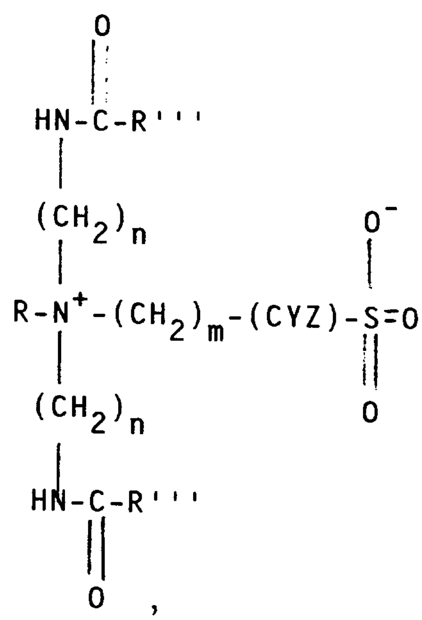

der Sarcosinate der allgemeinen Formel

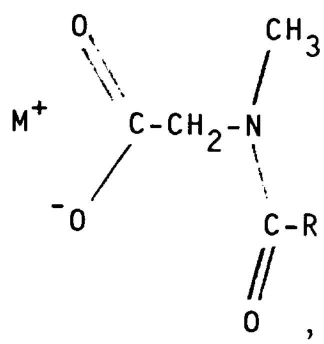

sowie der ethoxylierten Alkoholcarboxylate der allgemeinen Formel

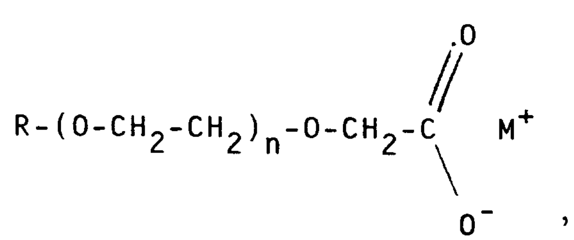

wobei

| | |
|---|---|
| R | einen gesättigten Kohlenwasserstoffrest mit 6 - 25 C-Atomen bedeutet, |
| R', R'' und R''' | unabhängig voneinander aus der Gruppe H, $-CH_2-CH_2-OH$ , $-(CH_2)_2COO^-$ $M^+$ gewählt werden, |
| Y, Z | unabhängig voneinander gewählt werden aus der Gruppe H, Methyl, |
| $M^+$ | ein Kation, gewählt aus der Gruppe $H^+$, $Na^+$, $K^+$, $NH_4^+$, $H_mN^+(-CH_2-CH_2-OH)_{4-m}$ , $H_mN^+(-CH_2-CH_3)_{4-m}$ oder $H_mN^+-(CH_3)_{4-m}$ bedeutet, |
| n | eine Zahl von 1 - 5 bedeutet, |
| m | 0, 1, 2 oder 3 bedeutet. |

**2.** Reinigungsmittel nach Anspruch 1, enthaltend, bezogen auf die Gesamtzusammensetzung
0,5 - 8 Gew.-% eines oder mehrerer Sulfosuccinate,
5 - 15 Gew.-% eines oder mehrerer Betaine
2 - 20 Gew.-% eines oder mehrerer Sarcosinate
10 - 20 Gew.-% eines oder mehrerer ethoxylierter Alkoholcarboxylate.

**3.** Reinigungsmittel nach Anspruch 1 oder 2, dadurch gekennzeichnet, daß das oder die Amidoamin-amphoteren Tenside gewählt werden aus der Gruppe der höheren Alkylamphodiacetate, der höheren Alkylamphoacetate, der höheren Alkylamphodipropionate, der höheren Alkylamphopropionate.

**4.** Reinigungsmittel nach einem der Ansprüche 1 - 3, dadurch gekennzeichnet, daß das oder die Amidoamin-amphoteren Tenside gewählt werden aus der Gruppe
Cocoamphodiacetate, Cocoamphoacetate, Cocoamphopropionate, Cocoamphodipropionate, Lauroamphodiacetate, Lauroamphoacetate, Lauroamphodipropionate, Caproamphodiacetate, Caproamphoacetate, Capryloamphodiacetate, Capryloamphoacetate, Capryloamphodipropionate, Stearoamphodiacetate, Stearoamphoacetate, Isostearoamphoacetate, Isostearoamphodipropionate.

**5.** Reinigungsmittel nach einem der Ansprüche 1 - 4 , dadurch gekennzeichnet, daß das oder die Sulfoacetat-Tenside gewählt werden aus der Gruppe
Laurylsulfoacetat, Cocoylsulfoacetat, Oleylsulfoacetat, Stearylsulfoacetat und Isostearylsulfoacetat.

**6.** Reinigungsmittel nach einem der Ansprüche 1 - 5 , dadurch gekennzeichnet, daß das oder die Betaine gewählt werden aus der Gruppe der höheren Alkyl-betaine, der Sulfo-betaine oder der Amidosulfobetaine.

**7.** Reinigungsmittel nach einem der Ansprüche 1 - 6 , dadurch gekennzeichnet, daß das oder die Betaine gewählt werden aus der Gruppe
Coco-dimethyl-carboxymethyl-betain,
Lauryl-carboxymethyl-betain,
Lauryl-dimethyl-(alpha-carboxyethyl)-betain,
Cetyl-dimethyl-carboxymethyl-betain,
Lauryl-(bis-(2-hydroxyethyl))-carboxymethyl-betain,
Stearyl-(bis-(2-hydroxypropyl))-carboxymethyl-betain,
Oleyl-dimethyl-(gamma-carboxypropyl)-betain,
Lauryl-(bis-(2-hydroxypropyl)-(alpha-carboxymethyl)-betain,
Coco-dimethyl-sulfodimethyl-sulfoethyl-betain,
Lauryl-(bis-(2-hydroxyethyl))-sulfopropyl-betain,
Coco-amidopropyl-betain, Cocobetain,
Myristyl-amidopropyl-betain.

**8.** Reinigungsmittel nach einem der Ansprüche 1 - 7 , dadurch gekennzeichnet, daß das oder die Sulfosuccinate gewählt werden aus der Gruppe der N-acyl-sulfosuccinate, bevorzugt gewählt aus der Gruppe
der Lauryl-sulfosuccinate, der Cocoyl-sulfosuccinate, der Oleyl-sulfosuccinate, der Stearyl-sulfosuccinate, der Isostearyl-sulfosuccinate.

**9.** Reinigungsmittel nach einem der Ansprüche 1 - 8 , dadurch gekennzeichnet, daß das oder die carboxylierten Alkoholethoxylate gewählt werden aus der Gruppe
Natrium-cocoylcarboxyamid-(MEA-4)-carboxylat,
Natrium-cocoylcarboxyamid-(MEA-3)-carboxylat,
Natrium-lauroylcarboxyamid-(MEA-4)-carboxylat,
Natrium-lauroylcarboxyamid-(MEA-3)-carboxylat,

**10.** Reinigungsmittel nach einem der Ansprüche 1 - 9 , enthaltend
2 - 8 Gew.-% eines oder mehrerer Amidoamin-amphoterer Tenside
0,5 - 5 Gew.-% eines oder mehrerer Sulfoacetat-Tenside und
0,5 - 5 Gew.-% eines oder mehrerer Sulfosuccinate,
5 - 12 Gew.-% eines oder mehrerer Betaine

2 - 10 Gew.-% eines oder mehrerer Sarcosinate
10 - 15 Gew.-% eines oder mehrerer carboxylierter Alkoholethoxylate.
mit der Maßgabe, daß die Gesamtmenge der Tenside höchstens 50 Gew.-%, bezogen auf das Gesamtgewicht der Zusammensetzung beträgt.

## Claims

**1.** Cleansing agents, preferably for cosmetic purposes, comprising
2 - 10% by weight of one or more amidoamineamphoteric surfactants,
0.5 - 15% by weight of one or more sulphoacetate surfactants and
if appropriate one or more surfactants chosen from the group comprising sulphosuccinates of the general formula

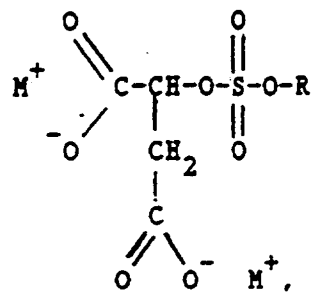

betaines of the general formula

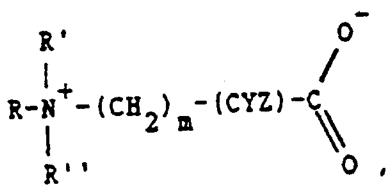

sulphobetaines of the general formula

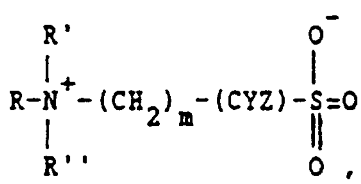

amidosulphobetaines of the general formula

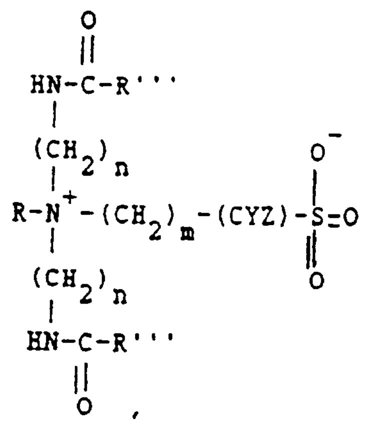

sarcosinates of the general formula

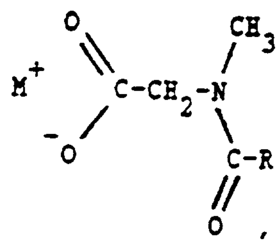

and ethoxylated alcohol carboxylates of the general formula

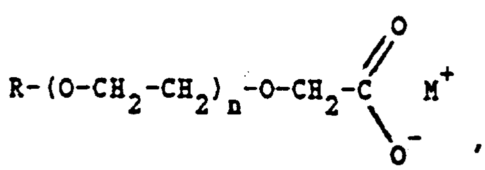

wherein

| | |
|---|---|
| R | denotes a saturated hydrocarbon radical having 6-25 carbon atoms, |
| R', R'' and R''' | are chosen independently of one another from the group H, $-CH_2-CH_2-OH$ and $-(CH_2)_2COO^-M^+$, |
| Y and Z | are chosen independently of one another from the group comprising H and methyl, |
| $M^+$ | denotes a cation chosen from the group comprising $H^+$, $Na^+$, $K^+$, $NH_4^+$, $H_mN^+(-CH_2-CH_2-OH)_{4-m}$, $H_mN^+(-CH_2-CH_3)_{4-m}$ and $H_mN^+-(CH_3)_{4-m}$, |
| n | denotes a number from 1 to 5 and |
| m | denotes 0, 1, 2 or 3. |

**2.** Cleansing agents according to Claim 1, comprising, based on the total composition,
0.5 - 8% by weight of one or more sulphosuccinates,
5 - 15% by weight of one or more betaines,
2 - 20% by weight of one or more sarcosinates and
10 - 20% by weight of one or more ethoxylated alcohol carboxylates.

**3.** Cleansing agents according to Claim 1 or 2, characterised in that the amidoamineamphoteric surfactant or surfactants are chosen from the group comprising higher alkyl amphodiacetates, higher alkyl amphoacetates, higher alkyl amphodipropionates and higher alkyl amphopropionates.

**4.** Cleansing agents according to one of Claims 1-3, characterised in that the amidoamineamphoteric surfactants are chosen from the group comprising coco-amphodiacetates, coco-amphoacetates, coco-amphopropionates, cocoamphodipropionates, lauro-amphodiacetates, lauro-amphoacetates, lauro-amophodipropionates, capro-amphodiacetates, capro-amphoacetates, capryloamphodiacetates, caprylo-amphoacetates, caprylo-amphodipropionates, stearo-amphodiacetates, stearo-amphoacetates, isostearo-amphoacetates and isostearo-amphodipropionates.

**5.** Cleansing agents according to one of Claims 1-4, characterised in that the sulphoacetate surfactant or surfactants are chosen from the group comprising lauryl sulphoacetate, (cocoyl)-sulphoacetate, oleyl sulphoacetate, stearyl sulphoacetate and isostearyl sulphoacetate.

**6.** Cleansing agents according to one of Claims 1-5, characterised in that the betaine or betaines are chosen from the group comprising higher alkyl-betaines, sulphobetaines and amidosulpho-betaines.

**7.** Cleansing agents according to one of Claims 1-6, characterised in that the betaine or betaines are chosen from the group comprising coconut-dimethyl-carboxymethylbetaine, lauryl-carboxymethyl-betaine, lauryl-dimethyl(alpha-carboxyethyl)-betaine, cetyl-dimethyl-carboxymethyl-betaine, lauryl-(bis-(2-hydroxyethyl)-carboxymethyl-betaine, stearyl-(bis-(2-hydroxypropyl))-carboxymethyl-betaine, oleyl-dimethyl-(gamma-carboxypropyl)-betaine, lauryl-(bis-(2-hydroxyypropyl)-(alpha-carboxymethyl)-betaine, coconut-dimethyl-sulphodimethyl-sulphoethyl-betaine, lauryl-(bis-(2-hydroxyethyl))-sulphopropyl-betaine, coconut-amidopropyl-betaine, coconut-betaine and myristyl-amidopropyl-betaine.

**8.** Cleansing agents according to one of Claims 1-7, characterised in that the sulphosuccinate or sulphosuccinates are chosen from the group comprising N-acyl sulphosuccinates, and are preferably chosen from the group comprising lauryl sulphosuccinates, (cocoyl)-sulphosuccinates, oleyl sulphosuccinates, stearyl sulphosuccinates and isostearyl sulphosuccinates.

**9.** Cleansing agents according to one of Claims 1-8, characterised in that the carboxylated alcohol ethoxylate or ethoxylates are chosen from the group comprising sodium (cocoyl)-carboxamide-(MEA-4)-carboxylate, sodium (cocoyl)-carboxamide-(MEA-3)-carboxylate, sodiumlauroyl-carboxamide-(MEA-4)-carboxylate and sodium lauroyl-carboxamide-(MEA-3)-carboxylate.

**10.** Cleansing agents according to one of Claims 1-9, comprising
2 - 8% by weight of one or more amidoamineamphoteric surfactants,
0.5 - 5% by weight of one or more sulphoacetate surfactants and
0.5 - 5% by weight of one or more sulphosuccinates,
5 - 12% by weight of one or more betaines,
2 - 10% by weight of one or more sarcosinates and
10 - 15% by weight of one or more carboxylated alcohol ethoxylates,
with the proviso that the total amount of surfactants is not more than 50% by weight, based on the total weight of the composition.

**Revendications**

**1.** Agents nettoyants, de préférence à but cosmétique, comprenant
2 - 10% en poids d'un ou de plusieurs tensioactifs amidoamines amphotères,
0,5 - 15% en poids d'un ou de plusieurs tensioactifs sulfoacétates, et
le cas échéant, un ou plusieurs des tensioactifs choisis dans le groupe comprenant les sulfosuccinates de formule générale

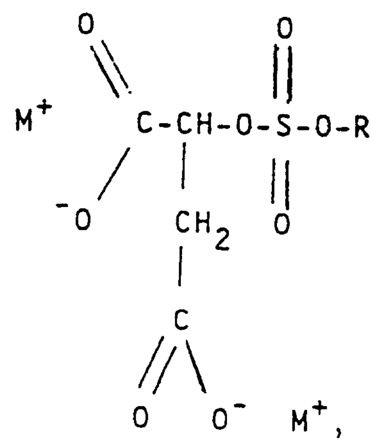

les bétaïnes de formule générale

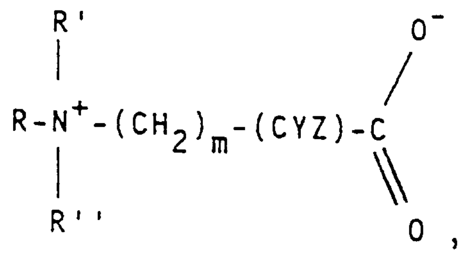

les sulfobétaïnes de formule générale

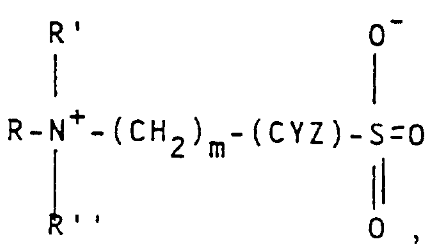

les amidosulfobétaïnes de formule générale

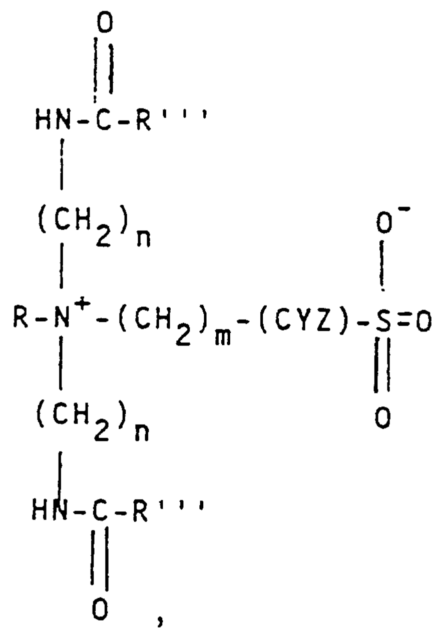

les sarcosinates de formule générale

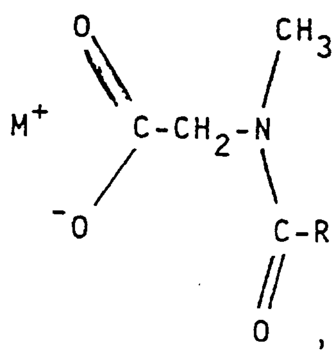

et les carboxylates d'alcools éthoxylés de formule générale

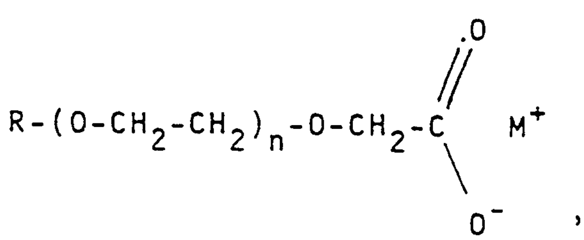

formules dans lesquelles

| | |
|---|---|
| R | représente un radical hydrocarboné saturé comportant 6-25 atomes de carbone, |
| R', R'' et R''' | sont choisis, indépendamment les uns des autres, dans le groupe comprenant H, $-CH_2-CH_2-OH$ et $-(CH_2)_2COO^-M^+$, |
| Y et Z | sont choisis, indépendamment l'un de l'autre, dans le groupe comprenant H et un groupe méthyle, |
| $M^+$ | représente un cation choisi dans le groupe comprenant $H^+$, $Na^+$, $K^+$, $NH_4^+$, $H_mN^+(-CH_2-CH_2-OH)_{4-m}$, $H_mN^+(-CH_2-CH_3)_{4-m}$ et $H_mN^+(CH_3)_{4-m}$, |
| n | représente un nombre de 1 à 5, et |

m représente 0, 1, 2 ou 3.

**2.** Agents nettoyants selon la revendication 1, comprenant, sur la base de la composition totale,
0,5 - 8% en poids d'un ou de plusieurs sulfosuccinates,
5 - 15% en poids d'une ou de plusieurs bétaïnes,
2 - 20% en poids d'un ou de plusieurs sarcosinates, et
10 - 20% en poids d'un ou de plusieurs carboxylates d'alcools éthoxylés.

**3.** Agents nettoyants selon la revendication 1 ou 2, caractérisés en ce que le ou les tensioactifs amidoamines amphotères sont choisis dans le groupe comprenant les amphodiacétates d'alkyle supérieur, les amphoacétates d'alkyle supérieur, les amphodipropionates d'alkyle supérieur et les amphopropionates d'alkyle supérieur.

**4.** Agents nettoyants selon l'une quelconque des revendications 1-3, caractérisés en ce que le ou les tensioactifs amidoamines amphotères sont choisis dans le groupe comprenant les amphodiacétates de coprah, les amphoacétates de coprah, les amphopropionates de coprah, les amphodipropionates de coprah, les lauro-amphodiacétates, les lauro-amphoacétates, les lauro-amphodipropionates, les capro-amphodiacétates, les capro-amphoacétates, les caprylo-amphodiacétates, les caprylo-amphoacétates, les caprylo-amphodipropionates, les stéaro-amphodiacétates, les stéaro-amphoacétates, les isostéaro-amphoacétates et les isostéaro-amphodipropionates.

**5.** Agents nettoyants selon l'une quelconque des revendications 1-4, caractérisés en ce que le ou les tensioactifs sulfoacétates sont choisis dans le groupe comprenant le sulfoacétate de lauryle, le sulfoacétate d'alkyle de coprah, le sulfoacétate d'oléyle, le sulfoacétate de stéaryle et le sulfoacètate d'isostéaryle.

**6.** Agents nettoyants selon l'une quelconque des revendications 1-5, caractérisés en ce que la ou les bétaïnes sont choisies dans le groupe comprenant les alkyl(supérieur)bétaïnes, les sulfo-bétaïnes et les amidosulfo-bétaïnes.

**7.** Agents nettoyants selon l'une quelconque des revendications 1-6, caractérisés en ce que la ou les bétaïnes sont choisies dans le groupe comprenant la diméthyl de coprah-carboxyméthyl-bétaïne, la lauryl-carboxyméthyl-bétaïne, la lauryl-diméthyl-(alpha-carboxyéthyl)-bétaïne, la cétyl-diméthyl-carboxyméthyl-bétaïne, la lauryl-(bis-(2-hydroxyéthyl))-carboxyméthyl-bétaïne, la stéaryl-(bis-(2-hydroxyypropyl))-carboxyméthyl-bétaïne, l'oléyl-diméthyl-(gamma-carboxypropyl)-bétaïne, la lauryl-(bis-(2-hydroxypropyl))-(alpha-[carboxyméthyl)-bétaïne, la diméthyl de coprah-sulfodiméthyl-sulfoéthyl-bétaïne, la lauryl-(bis-(2-hydroxyéthyl))-sulfopropyl-bétaïne, la coprah-amidopropyl-bétaïne, la coprah-bétaïne et la myristyl-amidopropyl-bétaïne.

**8.** Agents nettoyants selon l'une quelconque des revendications 1-7, caractérisés en ce que le ou les sulfosuccinates sont choisis dans le groupe comprenant les sulfosuccinates de N-acyle, et sont de préférence choisis dans le groupe comprenant les sulfosuccinates de lauryle, les sulfosuccinates d'alkyle de coprah, les sulfosuccinates d'oléyle, les sulfosuccinates de stéaryle et les sulfosuccinates d'isostéaryle.

**9.** Agents nettoyants selon l'une quelconque des revendications 1-8, caractérisés en ce que le ou les éthoxylats d'alcools carboxylés sont choisis dans le groupe comprenant l'alkylcarboxamide (de coprah)-(MEA-4)-carboxylate de sodium, l'alkylcarboxamide (de coprah)-(MEA-3)-carboxylate de sodium, le lauroylcarboxamide-(MEA-4)-carboxylate de sodium et le lauroylcarboxamide-(MEA-3)-carboxylate de sodium.

**10.** Agents nettoyants selon l'une quelconque des revendications 1-9, comprenant
2 - 8% en poids d'un ou de plusieurs tensioactifs amidoamines amphotères,
0,5 - 5% en poids d'un ou de plusieurs tensioactifs sulfoacétates et
0,5 - 5% en poids d'un ou de plusieurs sulfosuccinates,
5 - 12% en poids d'une ou de plusieurs bétaïnes,
2 - 10% en poids d'un ou de plusieurs sarcosinates, et
10 - 15% en poids d'un ou de plusieurs éthoxylats d'alcools carboxylés,

à condition que la quantité totale de tensioactifs ne dépasse pas 50% en poids, par rapport au poids total de la composition.